# EUROPEAN PATENT APPLICATION

(11) **EP 3 643 298 A1**
(43) Date of publication of application: **29.04.2020**
(21) Application number: 17914339.1
(22) Date of filing: 11.10.2017
(51) Int. Cl.: A61K 9/06, A61K 47/26, A61K 47/24, A61K 47/10, A61K 45/00, A61K 31/06

(54) **IN SITU PHASE CHANGE GEL SUSTAINED-RELEASE SYSTEM FOR SMALL MOLECULE DRUG AND PREPARATION METHOD THEREOF**

(30) Priority: 22.06.2017 CN 201710480564
(71) Applicant: Sichuan University, Chengdu, Sichuan 610065 (CN); Yaopharma Co., Ltd., Chongqing 401121 (CN)
(72) Inventor: GONG, Tao, Sichuan 610041 (CN); SONG, Xu, Sichuan 610041 (CN); ZHANG, Zhirong, Sichuan 610041 (CN); ZHANG, Yan, Chongqing 401121 (CN); WEI, Guangfei, Sichuan 610041 (CN); HU, Mei, Sichuan 610041 (CN); CHEN, Tijia, Sichuan 610041 (CN); SUN, Xun, Sichuan 610041 (CN); FU, Yao, Sichuan 610041 (CN)
(74) Representative: Schnappauf, Georg
(86) International application number: PCT/CN2017/105682
(87) International publication number: WO 2018/233148

(57) **Abstract**

An *in situ* phase change gel sustained-release preparation for a small molecule drug and a preparation method thereof. The *in situ* phase change gel sustained-release preparation comprises a small molecule drug comprising an active pharmaceutical ingredient, a phospholipid, Span, and an ethanol solution. The *in situ* phase change gel sustained-release preparation has a high concentration of phospholipids combined with Span, and is thus able to reduce the immediate-release of the small molecule drug and extend the release time, and is suitable for various administration routes, such as subcutaneous injection and external administration.

## Description

### CROSS REFERENCE TO RELATED APPLICATIONS

This application claims the priority to Chinese Patent Application No. 201710480564.3 titled *"IN SITU* PHASE CHANGE GEL SUSTAINED-RELEASE SYSTEM FOR SMALL MOLECULE DRUG AND PREPARATION METHOD THEREOF", filed with the China National Intellectual Property Administration on June 22, 2017, which is incorporated herein by reference in its entirety.

### FIELD

The present disclosure relates to an *in-situ* phase change gel sustained-release system suitable for small molecule drugs using phospholipid and Span as matrix and ethanol as solvent, belonging to the technical field of medicine.

### BACKGROUND

Small molecule drugs have an outstanding and irreplaceable important role in human health. They have advantages of small relative molecular weight, clear physicochemical properties, simple structure, good stability, low price, and no antigenicity, but also have some problems. The blood drug concentration after an administration of the small molecule drug has obvious peak and valley values, so that the toxic side effect is large and the drug effect decreases; for the chronic disease, a long-term continuous administration is required, which aggravates the physical, psychological and economic burden of the patient. The sustained release preparation can slowly release the drug to obtain a stable blood drug concentration, and reduce the peak-to-valley value, which is the key to attenuating toxicity and increasing the efficacy, and increasing the compliance of the patient. Therefore, it has been desired to develop a sustained release drug delivery system for injectable small molecule drugs.

Currently, the sustained release drug delivery system for injection is mainly PLGA (polylactic acid-glycolic acid copolymer) microspheres. Among the many small-molecule drug sustained release preparations, a risperidone sustained release microsphere for injection is one of the most successful products. Risperidone is one of the drugs for the treatment of psychosis. Long-acting risperidone microsphere for injection (abbreviated as "risperidone microspheres"), trade name Consta, was developed and manufactured by Janssen Pharmaceuticals in the United States, and it was marketed in the United States and Europe in 2003 and entered the Chinese market in 2006, which can be sustained released for 7 weeks. Luye Pharma Group has developed its own risperidone sustained release microsphere intramuscular injection preparation (LY3004) for the treatment of schizophrenia, and three key Phase I clinical trials have been completed in 172 US patients. The preparation only need to be injected once every two weeks, easy to use. Although the PLGA microspheres have a good sustained release effect, the preparation process is complicated, the drug loading is low, and the organic solvent used in the preparation process remains in the preparation, and the lactic acid and glycolic acid produced during the degradation process cause a decrease in the pH at the injection site, which may cause an inflammatory reaction. The above disadvantages limit the application of PLGA microspheres.

The vesicular phospholipid gel (VPG) is a semi-solid phospholipid dispersion system that can encapsulate water-soluble, fat-soluble and amphiphilic drugs. The patent CN102697741A has developed an oxaliplatin vesicular phospholipid gel injection prepared by oxaliplatin, soy lecithin, cholesterol, PEG and glucose in a specific weight ratio, thereby improving the stability of the preparation. However, it can be seen from the results that the preparation has no obvious advantage in prolonging the drug release time and inhibiting the burst release, and the release time is short. Moreover, problems such as sedimentation and aggregation which are prone to occur during the storage process, will affect the stability of the preparation and are not conducive to the preservation and transportation of the preparation.

The laboratory developed an *in-situ* phase change gel sustained release preparation (CN102526753A) with a high concentration of phospholipid as the main matrix with an addition of a small portion of vegetable oil. The phospholipid gel preparation has the advantages of good biocompatibility, remarkable sustained release effect, good degradability *in vivo,* and the like, and has good sustained release effect when applied to protein polypeptide drugs. For example, octreotide acetate can be smoothly sustained released in rats, rabbits and dogs for about one month, and the drug has little burst release, which is superior to commercially available octreotide acetate microspheres (MX Wang,et al. Pharmacokinetic and pharmacodynamic study of a phospholipid-based phase separation gel for once a month administration of octreotide, Journal of Controlled Release 230 (2016) 45-56); and exenatide acetate phospholipid gel has almost no burst release, which can be sustained released in rats for up to one month, and can maintain stable blood glucose for more than 20 days (M Hu, et al. Long-Acting Phospholipid Gel of Exenatide for Long-TermTherapy of Type II Diabetes, Pharm Res 33 (2016):1318 - 1326). The phospholipid gel preparation has a good sustained release effect and small burst release when applied to partially water-soluble small molecule chemical drugs, for example, doxorubicin hydrochloride and bromotetrandrine hydrochloride phospholipid gel can continue to be released in animals for more than half a month (JW Luo,et al. A novel injectable phospholipid gel co-loaded with doxorubicin and bromotetrandrine for resistant breast cancer treatment by intratumoral injection, Colloids and Surfaces B: Biointerfaces 140 (2016) 538-547). However, we also found in the study that for many small molecule chemical drugs entrapped with this type of phospholipid gel preparation, the effect is not ideal. Although there is some sustained release effect, but the sustained release time can only last for several days and a long-term effective blood drug concentration cannot be maintained, and the burst release is more serious. Especially for fat-soluble drugs, the burst release is obvious. The presumed reason may be that the phospholipid is a fat-soluble component while the high water soluble drug entrapped thereof is difficult to diffuse and the release of the drug mainly depends on the surface erosion of the phospholipid. Therefore, the duration of drug release is almost identical to the elimination time of the phospholipid carrier at the injection site *in vivo.* However, the fat-soluble drug has a good compatibility with the phospholipid, and is easily penetrated, diffused and released in the phospholipid carrier, resulting in a markedly accelerated release rate, a relatively short sustained release time, and a large burst release. For some drugs with narrow therapeutic windows, safety issues may be caused.

Therefore, for small molecule drugs, it is necessary to find a drug carrier with a wide application range, small burst release and long sustained release time to solve the problems of long-acting sustained release for injection.

### SUMMARY

The object of the present disclosure is to overcome the defects of the conventional art and to provide an *in-situ* gel sustained release system with a wide range of application, which can not only enable long-term sustained release of water-soluble small molecule drugs after injection, but also can be applied to fat-soluble small molecule drugs. The *in-situ* gel system may significantly reduce the burst release of small molecule drugs.

It was unexpectedly found in the study that a mixture of high concentrations of phospholipids and Span can form a flowing injectable liquid in ethanol, which is clear and transparent. When the mixture is poured into water, it can rapidly spontaneously form a semisolid gel preparation. According to this property, the inventors assume that after dissolving or dispersing the drug in an ethanol solution containing phospholipids and Spans, and injecting the mixture into the body, the phospholipid and the Span will spontaneously form a semisolid gel at the injection site due to the relatively high water content in the body. Simultaneously, with the exudation of ethanol, the mixture is further solidified to become a carrier for drug release and effectively control the release of the drug.

According to the above accidental discovery, the inventors thus use 2,4-dinitrophenol as a model drug and prepare an ethanol solution containing 30% Span 80 and 45% phospholipids. After 0.5 ml of subcutaneous injection in rats, the drug can be released smoothly for more than 10 days. At the same dose, the Cmax of the original drug ≈ 54.48 µg/mL, the Cmax of the high-concentration phospholipid gel (prepared according to patent CN102526753A) ≈ 30.28µg /mL, the Cmax of the Span phospholipid gel ≈ 14.86µg /mL, and the original drug group can only be released for 24h. The toxicity test results show that the phospholipid Span gel can effectively reduce the toxic side effects of 2,4-dinitrophenol. Thus, for a drug with a narrow therapeutic window such as 2,4-dinitrophenol, such preparation can effectively reduce its toxic side effects.

According to the above creative studies, we have surprisingly found that phospholipid Span gel sustained release preparations may significantly reduce the burst release of small molecule drugs and prolong the release time. We believe that the new gel sustained release system prepared herein can reduce burst release and prolong sustained release time. Therefore, we selected dabigatran etexilate and brexpiprazole as model drugs for further studies, and the results showed that dabigatran etexilate and brexpiprazole gel preparations have been in a stable release state with almost no burst release.

Span is a nonionic surfactant with low hydrophilic-lipophilic balance (HLB value) and it has good hydrophobicity and safety. The inventors have found that when some Span is added to the preparation, the gel preparation is solidified immediately upon contact with water. On the one hand, the solidification time of the phospholipid gel can be shortened; on the other hand, the small molecule chemical drug has a good sustained release effect in the *in vitro* and *in vivo* release test, thereby expanding the application of the phospholipid gel preparation, and providing a possibility for the sustained release application of the small molecule drug.

In addition, this type of phospholipid gel still has a good sustained release effect when applied to water-soluble small molecule drugs, such as doxorubicin hydrochloride.

One of the objects of the present disclosure is to provide an *in-situ* gel sustained release system suitable for use in small molecule drugs.

One of the objects of the present disclosure is to provide an *in-situ* gel sustained release system which can significantly reduce the burst release of a fat-soluble small molecule drug.

One of the objects of the present disclosure is to provide a composition comprising a phospholipid, a Span, and an ethanol solution.

One of the objects of the present disclosure is to provide a high-concentration phospholipid Span sustained release preparation prepared from a phospholipid, a Span, and an ethanol solution, that is, the *in-situ* injection phase change gel sustained release system or an *in-situ* injection phase change gel sustained release preparation according to the present disclosure.

One of the objects of the present disclosure is to provide a phospholipid Span sustained release preparation containing a biologically active ingredient, which has a good sustained release effect, effectively reduces drug release, has a high concentration of phospholipid, and is easy to inject.

The Span suitable for use in the phospholipid Span sustained release preparation of the present disclosure comprises, but is not limited to, one or more of Span 80, Span 85, Span 60, Span 40, Span 20.

In a specific embodiment, Span 80 is preferred according to the present disclosure.

The phospholipid suitable for use in the high-concentration phospholipid Span sustained release preparation of the present disclosure comprises, but is not limited to, one or more of natural phospholipids, semisynthetic phospholipids, and synthetic phospholipids.

The natural phospholipid comprises, but is not limited to, egg yolk lecithin, soybean lecithin, or combinations thereof.

The semisynthetic phospholipid comprises, but is not limited to, hydrogenated egg yolk lecithin, hydrogenated soybean lecithin, or combinations thereof.

The synthetic phospholipid comprises, but is not limited to, one or more of dipalmitoyl phosphatidylethanolamine, dipalmitoyl phosphatidic acid, dipalmitoyl phosphatidylglycerole, dioleoyl phosphatidylethanolamine, dipalmitoyl phosphatidylcholine, distearoyl phosphatidylcholine, dimyristoyl phosphatidylcholine.

In a specific embodiment, soybean lecithin S100 is preferred according to the present disclosure.

In a specific embodiment, the phospholipid sustained release preparation of the present disclosure has a phospholipid content of about 40% - 60% (g/g), and a Span content of less than 40% (g/g) based on the total weight of the preparation.

The pharmaceutically active ingredient suitable for preparing the phospholipid sustained release preparation of the present disclosure may be a water-soluble molecule, a fat-soluble molecule or an amphiphilic molecule, including but not limited to an anticancer drug, an anti-inflammatory drug, an analgesic drug, an anti-infective drug, an anti-diabetic drug, an anti-immune drug, an antihypertensive drug, an anti-epileptic drug, an anti-depressive drug, an antipsychotic drug, an anti-obesity drug, a drug for treating urinary tract diseases, and an cardiotonic drug.

The pharmaceutically active ingredient suitable for use in the present disclosure comprises, but is not limited to one or more of 2,4-dinitrophenol, dabigatran etexilate, aspirin, ridogrel, ticlopidine, clopidogrel, heparin, logiparin, lomoparin, warfarin, dicoumarol, theophylline,aminophylline, choline theophyllinate, salbutamol, clenbuterol, terbutaline, ipratropium bromide, sodium tryptophan, ketotifen, sodium valproate, carbamazepine, phenytoin sodium, ethosuximide, primidone, chloral hydrate, zopiclone, zaleplon, phenobarbital, amobarbital, sodium thiopental, diazepam, oxazepam, clonazepam, nitrazepam, triazolam, alprazolam, estazolam, glipizide, diltiazem, tramadol, morphine, pethidine, fentanyl, methadone, pentazocine, buprenorphine, codeine, naloxone, diclofenac, diclofenac sodium, ibudilast, ambroxol hydrochloride, oxycodone, chlorpromazine, trifluoperazine, fluphenazine, fluphenazine decanoate, fluphenazine enanthate, perphenazine enanthate, perphenazine, thioridazine, chlorprothixene , flupentixol decanoate, clopenthixlo, haloperidol, haloperidol decanoate, pipotiazine palmitate, trifluridol, pimozide, trichlorpromazine, thioridazine, fluspirilene, droperidol, clozapine, loxapine, clotiapine, olanzapine, quetiapine, sulpiride, sultopride, tiapride, tiapride, penfluridol, risperidone, morpholone, oxypertine, clomacran, reserpine, imipramine, amitriptyline, maprotiline, clomipramine, mianserin, sertraline, fluoxetine, fluvoxamine, citalopram, moclobemide, trazodone, lithium carbonate, tolterodine, levodopa, ziprasidone, levetiracetam, retigabine, perampanel, brivaracetam, eslicarbazepine, oxcarbazepine, duloxetine, paroxetine, vortioxetine, desmethylvenlafaxine, escitalopram, agomelatine, reboxetine, cariprazine, paliperidone, pramipexole, rasagiline, ropinirole, opicapone, safinamide, alfacalcidol, eldecalcitol, edaravone, aripiprazole, brexpiprazole, carbidopa, benserazide, selegiline, amantadine, memantine, bromocriptine, trihexyphenidyl, galantamine, huperzine A, rivastigmine, xanomeline, citicoline, piracetam, pyritinol, venlafaxine, bupropion, fluvastatin sodium, gliclazide, metformin, acyclovir, bezafibrate, fenofibrate,gemfibrozil, ciprofibrate, niacin, allopurinol, magnesium valproate, vincamine, promethazine, diphenhydramine, tripelennamine, chlorpheniramine, buclizine, phenindamine, cyproheptadine, hydroxyzine, cyclizine, meclizine, loratadine, cetirizine, efletirizine, metronidazole, clemastine, azelastine, acrivastine, mizolastine, astemizole, pheniramine, brompheniramine, tolpropamid, pyrrobutamine, triprolidine, nefopam, indapamide, tamsulosin, emedastine, oxybutynin, buflomedil, tamsulosin, propranolol, metoprolol,nadolol, pindolo, atenolol, alprenolol, acebutolol, bisoprolol, betaxolol, labetalol, carazolol, prazosin, captopril, enalapril, benazepril, fosinopril, cilazapril, trandolapril, alacepril, delapril, perindopril, quinapril, nifedipine, amlodipine, levoamlodipine, nimodipine, nicardipine, felodipine, lacidipine, nisoldipine, isradipine, verapamil, losartan, valsartan, telmisartan, irbesartan, candesartan, pomisaratan, terazosin, doxazosin, clonidine, moxonidine, reserpine, guanethidine, sodium nitroprusside, hydralazine, minoxidil, pinacidil, nicorandil, urapidil, piribedil, cicletanine, enalkiren, remikiren, hydrochlorothiazide, bendroflumethiazide, hydroflumethiazido, cyclopenthiazide, curcumin, tacrine, almitrine, platinum ligand, methotrexate, fluorouracil, thiopurine, tioguanine, hydroxyurea, cytarabine, everolimus, tacrolimus, acipimox, sirolimus, tegafur, pentostatin, gemcitabine, cyclophosphamide, busulfan, carmustine, dacarbazine, vinblastine, vincristine, vinorelbine, vindesine, paclitaxel, docetaxel, cabazitaxel, camptothecin, hydroxycamptothecin, teniposide, etoposide, irinotecan, topotecan, harringtonine, homoharringtonine, doxorubicin, pirarubicin, aclarubicin, idarubicin, epirubicin, daunorubicin, actinomycin D, plicamycin, clarithromycin, mitoxantrone, ibuprofen, acetaminophen, sulindac, fenoprofen, flurbiprofen, ketoprofen, meloxicam, piroxicam, nimesulide, rofecoxib, celecoxib, colchicine, probenecid, sulfinpyrazone, benzbromarone, phenylbutazone, mefenamic acid, clofenamic acid, naproxen, indomethacin, etodolac, magnesium salicylate, choline salicylate, salicylamide, salsalate, diflunisal, adrenocortical hormone, estrogen, androgen, tamoxifen, raloxifene, clomiphene, methyltestosterone, testosterone propionate, testosterone phenylacetate, medroxyprogesterone, megestrol, chlormadinone, hydroxyprogesterone acetate, hydrocortisone, cortisone, prednisone, prednisolone, methylprednisolone, dexamethasone, betamethasone, flutamide, retinoic acid, imatinib, gefitinib, erlotinib, thalidomide, lenalidomide, procarbazine, chlorambucil, melphalan or a pharmaceutically acceptable salt of the above medicaments. A fat-soluble drug is preferred.

Further, the pharmaceutically acceptable salt forms of the above chemical agents include, but are not limited to, various pharmaceutically usable salt forms such as hydrochloride, sulfate, acetate, salicylate, sulfonate, and citrate.

The phospholipid Span sustained release preparation according to the present disclosure comprises, in parts by weight, 0.01-20 parts of pharmaceutically active ingredient, 30-60 parts of phospholipid, 10-40 parts of Span, and 7-30 parts of ethanol solution.

Further, in percentages by weight, the phospholipid accounts for 40%-60% of the content of the phospholipid Span sustained release preparation, and the Span accounts for less than 40% of the content of the phospholipid Span sustained release preparation.

Among them, the ethanol solution comprises ethanol-water solution, ethanol-normal saline solution, ethanol-phosphate buffer solution, ethanol carbonate buffer solution, ethanol-succinate buffer solution, ethanol-citrate buffer solution, and ethanol-lactate buffer solution, wherein the concentration of the ethanol may be 70%-100% (v/v).

The phospholipid Span sustained release preparation of the present disclosure may be added with a conventional excipient which is used in pharmacy such as an antioxidant, a preservative, a pH adjuster or the like.

The *in-situ* injection phase change gel sustained release system using the phospholipid Span as a matrix according to the present disclosure is a novel dosage form, and the active pharmaceutical ingredient can be dissolved in the phospholipid Span-ethanol solution to become a liquid with good fluidity. After injection into the body, the phospholipid and the Span will immediately form a semisolid gel encapsulating the active pharmaceutical ingredient, and the gel have a good sustained release effect.

The active pharmaceutical ingredient may also be dispersed in the form of microparticles in a phospholipid Span-ethanol solution as long as the preparation has the ability of passing through the needle pinhole.

The active pharmaceutical ingredient and the blank phospholipid Span matrix can be separately stored, and dissolved or dispersed uniformly before administration.

Another object of the present disclosure is to provide a method for preparing a phospholipid Span gel sustained release system, i.e., a phospholipid Span sustained release preparation.

The preparation method according to the disclosure comprising the following steps
(1) dissolving an active pharmaceutical ingredient in an appropriate amount of ethanol solution, filtering and sterilizing by a microporous membrane to form a drug solution; and
(2) mixing an injection grade phospholipid and a Span with the drug solution of step (1) under a sterile condition, stirring to completely dissolve the phospholipid, leaving to stand for a moment to remove bubbles in the preparation, subpackaging and sealing.

Another specific embodiment of the present disclosure also provides another method for preparing a phospholipid Span sustained release preparation, which can help poor soluble active pharmaceutical ingredients to obtain uniformly dispersed preparations. The method comprises the following steps
(1) subjecting an active pharmaceutical ingredient to a pharmaceutically common crystallization or pulverization under a sterile condition to prepare drug microparticles;
(2) mixing a prescription amount of phospholipid and Span with an ethanol solution under a sterile condition, stirring to completely dissolve the mixture to prepare a carrier solution; and
(3) mixing the drug microparticles of step (1) with the carrier solution prepared in step (2) uniformly under a sterile condition, leaving to stand for a moment to remove bubbles in the preparation, subpackaging and sealing.

In a specific embodiment, the microporous membrane is preferably a 0.22µm microporous membrane.

The *in-situ* injection phase change gel sustained release preparation according to the present disclosure can be used for injection administration, preferably subcutaneous administration; and can also be used in other administration forms such as external administration.

The *in-situ* injection phase change gel sustained release preparation according to the present disclosure can also be used to repair and augment soft and/or hard tissues.

Through creative research, the present disclosure, using a phospholipid Span and an ethanol solution as main raw materials, and using small molecule chemical drugs such as 2,4-dinitrophenol, dabigatran etexilate, and brexpiprazole as model drugs to prepare a phospholipid Span sustained release preparation by simple stirring. The obtained preparation has good fluidity and is easy to be administered by injection. Animal experiments *in vivo* have shown that it has a good sustained release effect. By investigating the irritation of the phospholipid Span sustained release preparation at the injection site, it is showed that the preparation has good biocompatibility and only cause weak irritation at the administration site.

Therefore, the present disclosure can generally realize the sustained release of the small molecule drug, and well solves the problem of strong burst release of the existing small molecule drug *in-situ* gel preparation, and has a good application prospect.

### ADVANTAGES OF PRESENT DISCLOSURE

The phospholipid Span sustained release preparation contains a small amount of ethanol solution which is completely miscible with water. After injection into the body, the mixture of phospholipid and Span can be immediately solidified, and the ethanol can rapidly diffuse into the body fluid to further solidify the gel preparation, which becomes a carrier of the sustained release of the drug and control the release of the drug. The *In vivo* experiments in animals have shown that the preparation does have a good sustained release effect, and the burst release effect is weak or even no burst release. The phospholipid Span sustained release preparation has advantages such as good fluidity and being easy to inject. Besides, it is also possible to selecte an appropriate solvent or a combination of solvents according to the solubility of the drug to be entrapped to dissolve a drug, to obtain a uniformly transparent stable preparation. The drug may also be uniformly dispersed in the preparation in the form of microparticles. In addition, the phospholipid Span sustained release preparation contains no water or has a low water content, and it contains ethanol, which can effectively inhibit the growth of microorganisms and facilitate the storage of the preparation, thereby expanding the application range of the phospholipid Span preparation.

### BRIEF DESCRIPTION OF DRAWINGS

Figure 1 is a process diagram showing formation of a gel from a phospholipid Span sustained release preparation.
Figure 2 shows an *in vitro* release profile of a phospholipid Span sustained release preparation (DNP-LC-gel) of 2,4-dinitrophenol.
Figure 3 shows a drug concentration-time curve of a phospholipid Span sustained release preparation, a high-concentration phospholipid sustained release preparation, and a solution group of 2, 4-dinitrophenol.
Figure 4 shows a drug concentration-time curve of a phospholipid Span sustained release preparation of 2,4-dinitrophenol for subcutaneous injection.
Figure 5 shows a toxicity test of a phospholipid Span sustained release preparation of 2,4-dinitrophenol.
Figure 6 shows a drug concentration-time curve of a phospholipid Span sustained release preparation, a high-concentration phospholipid sustained release preparation, and a solution group of dabigatran etexilate.
Figure 7 shows a local irritation test of a phospholipid sustained release preparation of dabigatran etexilate.
Figure 8 shows a drug concentration-time curve of a phospholipid Span sustained release preparation of brexpiprazole and F127 sustained release preparation.

### DETAILED DESCRIPTION

The present disclosure discloses a small molecule drug *in-situ* phase change gel sustained release system and a preparation method thereof. Those skilled in the art can learn from the contents of the present disclosure and appropriately improve the process parameters. It is to be noted that all such alternatives and modifications are obvious to those skilled in the art and are considered to be included in the present disclosure. The method and the application of the present disclosure have been described by the preferred embodiments, and those skilled in the art can obviously modify or appropriately change and combine the method and application described herein without departing from the spirit and scope of the present disclosure, to implement and apply the techniques of the present disclosure.

The granule composition, and the raw material or excipients used in the preparation method or preparation thereof according to the present disclosure are commercially available.

The present disclosure is further illustrated below in conjunction with the examples.

### Example 1

50mg of 2,4-dinitrophenol was dissolved in 2.0g of 85% (v/v) ethanol-pH7.6 phosphate buffer. The mixture was then filtered by 0.22µm microporous membrane to obtain a drug solution. Then 4.5g of injection grade soy lecithin S100 and 3.5g of Span 80 were added under a sterile condition. The mixture was magnetically stirred for about 1h under the sterile condition, so that S100 was completely dissolved to obtain a liquid containing a large number of bubbles, and the liquid was left to stand until the bubbles completely disappeared. The liquid was subpackaged and sealed to obtain the product.

### Example 2

200mg of dabigatran etexilate was dissolved in 1.0g of anhydrous ethanol to obtain a drug solution, which was filtered by 0.22µm microporous membrane. Then 5.0g of injection grade egg yolk lecithin E80 and 4.0g of Span 80 were added under a sterile condition. The mixture was magnetically stirred for about 1h under the sterile condition, so that E80 was completely dissolved to obtain a liquid containing a large number of bubbles, and the liquid was left to stand until the bubbles completely disappeared. The liquid was subpackaged and sealed to obtain the product.

### Example 3

Under a sterile condition, an appropriate amount of brexpiprazole was ground and pulverized in a mortar to obtain a brexpiprazole powder. 1.5g of anhydrous ethanol, 4.0g of Span 80 and 4.5g of injection grade soy lecithin S100 were magnetically stirred under the sterile condition for about 1h, so that S100 was completely dissolved to obtain a creamy yellow liquid containing a large number of bubbles. 200mg of brexpiprazole powder was mixed uniformly under the sterile condition with the above liquid, which was left to stand until the bubbles completely disappeared. The liquid was subpackaged and sealed to obtain the product.

### Example 4

100mg of theophylline was dissolved in 3.0g of 80% (v/v) ethanol-pH7.6 phosphate buffer to obtain a drug solution, which was then filtered by 0.22µm microporous membrane. Then 4.0g of injection grade hydrogenated egg yolk lecithin and 1.0g of Span 80 and 1.0g of Span 20 were added under a sterile condition. The mixture was magnetically stirred for about 1h under the sterile condition, so that hydrogenated egg yolk lecithin was completely dissolved to obtain a liquid containing a large number of bubbles, and the liquid was left to stand until the bubbles completely disappeared. The liquid was subpackaged and sealed to obtain the product.

### Example 5

10.0mg of dexamethasone was dissolved in 0.7g of anhydrous ethanol to obtain a drug solution which was filtered by 0.22µm microporous membrane. Then 6.0g of injection grade dipalmitoyl phosphatidylethanolamine, 2.3g of Span 20, and 1.0g of Span 60 were added under a sterile condition. The mixture was magnetically stirred for about 1h under the sterile condition, so that the dipalmitoyl phosphatidylethanolamine was completely dissolved to obtain a liquid containing a large number of bubbles, and the liquid was left to stand until the bubbles completely disappeared. The liquid was subpackaged and sealed to obtain the product.

### Example 6

100mg of aspirin was dissolved in 2.0g of 80% (v/v) ethanol-pH7.6 phosphate buffer to obtain a drug solution, which was filtered by 0.22µm microporous membrane. Then 4.0g of injection grade egg yolk lecithin E80 and 3.9g of Span 20 were added under a sterile condition. The mixture was magnetically stirred for about 1h under the sterile condition, so that E80 was completely dissolved to obtain a liquid containing a large number of bubbles, and the liquid was left to stand until the bubbles completely disappeared. The liquid was subpackaged and sealed to obtain the product.

### Example 7

200mg of cimetidine was dissolved in 2.8g of 90% (v/v) ethanol-pH7.6 phosphate buffer to obtain a drug solution, which was filtered by 0.22µm microporous membrane. Then 6.0g of injection grade soy lecithin S100 and 1.0g of Span 20 were added under a sterile condition. The mixture was magnetically stirred for about 1h under the sterile condition, so that S100 was completely dissolved to obtain a liquid containing a large number of bubbles, and the liquid was left to stand until the bubbles completely disappeared. The liquid was subpackaged and sealed to obtain the product.

### Example 8

40mg of donepezil hydrochloride was dissolved in 2.5g of 70% (v/v) ethanol-water to obtain a drug solution, which was filtered by 0.22µm microporous membrane. Then 6.0g of injection grade soy lecithin S100, 1.0g of Span 80, and 0.5g of Span 40 were added under a sterile condition. The mixture was magnetically stirred for about 0.5h under the sterile condition, so that S100 was completely dissolved to obtain a liquid containing a large number of bubbles, and the liquid was left to stand until the bubbles completely disappeared. The liquid was subpackaged and sealed to obtain the product.

### Example 9

Under a sterile condition, an appropriate amount of risperidone was ground and pulverized in a mortar to obtain a risperidone powder. 2.0g of anhydrous ethanol solution, 3.5g of Span 20 and 4.5g of soy lecithin S100 were magnetically stirred under the sterile condition for about 0.5h, so that S100 was completely dissolved to obtain a creamy yellow liquid containing a large number of bubbles. 150mg of risperidone powder was mixed uniformly under the sterile condition with the above liquid, which was left to stand until the bubbles completely disappeared. The liquid was subpackaged and sealed to obtain the product.

### Example 10

200mg of captopril was dissolved in 1.5g of 90% (v/v) ethanol-water solution to obtain a drug solution, which was filtered by 0.22µm microporous membrane. Then 5.5g of injection grade egg yolk lecithin E80 and 3.0g of Span 85 were added under a sterile condition. The mixture was magnetically stirred for about 0.5h under the sterile condition, so that E80 was completely dissolved to obtain a creamy yellow liquid containing a large number of bubbles, and the liquid was left to stand until the bubbles completely disappeared. The liquid was subpackaged and sealed to obtain the product.

### Example 11

100mg of diazepam was dissolved in 2.0g of 75% (v/v) ethanol-water to obtain a drug solution, which was filtered by 0.22µm microporous membrane. Then 5.5g of injection grade egg yolk lecithin E80 and 2.5g of Span 20 were added under a sterile condition. The mixture was magnetically stirred for about 0.5h under the sterile condition, so that E80 was completely dissolved to obtain a creamy yellow liquid containing a large number of bubbles, and the liquid was left to stand until the bubbles completely disappeared. The liquid was subpackaged and sealed to obtain the product.

### Example 12

80mg of promethazine was dissolved in 2.0g of 70% (v/v) ethanol-water to obtain a drug solution, which was filtered by 0.22µm microporous membrane. Then 5.5g of soy lecithin S100 and 3.5g of Span 80 were added under a sterile condition. The mixture was magnetically stirred for about 0.5h under the sterile condition, so that S100 was completely dissolved to obtain a creamy yellow liquid containing a large number of bubbles, and the liquid was left to stand until the bubbles completely disappeared. The liquid was subpackaged and sealed to obtain the product.

### Example 13

70mg of aclarubicin was dissolved in 2.0g of 80% (v/v) ethanol-water to obtain a drug solution, which was filtered by 0.22µm microporous membrane. Then 5.5g of egg yolk lecithin E80 and 2.5g of Span 80 were added under a sterile condition. The mixture was magnetically stirred for about 0.5h under the sterile condition, so that E80 was completely dissolved to obtain a creamy yellow liquid containing a large number of bubbles, and the liquid was left to stand until the bubbles completely disappeared. The liquid was subpackaged and sealed to obtain the product.

### Example 14

90mg of carmustine was dissolved in 2.5g of 80% (v/v) ethanol-water to obtain a drug solution, which was filtered by 0.22µm microporous membrane. Then 5.5g of soy lecithin S100 and 2.0g of Span 80 were added under a sterile condition. The mixture was magnetically stirred for about 0.5h under the sterile condition, so that S100 was completely dissolved to obtain a creamy yellow liquid containing a large number of bubbles, and the liquid was left to stand until the bubbles completely disappeared. The liquid was subpackaged and sealed to obtain the product.

### Experimental Example 1

The following test was carried out in accordance with the preparation method of Example 1, except that the Span in the prescription of Example 1 was replaced with glyceryl dioleate (Test Example 1), Tween (Test Example 2) and sucrose acetate isobutyrate (Test Example 3). At the same time, the original drug solution group was designed (Test Example 4, 50 mg of the drug was dissolved in 10 mL of phosphate buffer (pH=7.6), and a solution of the same concentration as in Example 1 was prepared).

200-220g of SD rats were randomly divided into five groups, 6 rats in each group, and were administered subcutaneously with 12.5 mg/kg dosage of 2,4-dinitrophenol (Example 1, Test Example 1, Test Example 2 , Test Example 3 and Test Example 4). At the scheduled time point, blood was taken from the rat eye and placed in a heparin sodium anticoagulated test tube. After the sample was processed, the blood concentration was measured by LC-MS/MS. The results are shown in the following table:

| Groups | Surfactant | Dosage (g) | Cₘₐₓ(µg/mL) |
|---|---|---|---|
| Example 1 | Span 80 | 3.5 | 14.86 |
| Test Example 1 | glyceryl dioleate | 3.5 | 25.42 |
| Test Example2 | Tween | 3.5 | 30.65 |
| Test Example3 | sucrose acetate isobutyrate | 3.5 | 28.74 |
| Test Example4 | Original drug solution (5 mg/mL) | | 54.48 |

Results: it can be seen from the above table that the Span, which is added to the phospholipid gel, is superior to other surfactants, and can effectively inhibit the burst release effect of 2,4-dinitrophenol.

### Experimental Example 2

The following test was carried out in accordance with the preparation method of Example 1, except that the ethanol in the prescription of Example 1 was replaced with propylene glycol (Test Example 1) and glycerin (Test Example 2). The appearance of the preparation was examined and the viscosity was measured by a viscometer.

The results are shown in the following table:

| Groups | Solvent | Dosage (g) | Viscosity (cp) |
|---|---|---|---|
| Example 1 | ethanol | 2.0 | 210 |
| Test Example 1 | propylene glycol | 2.0 | S100 cannot be dissolved with overnight stirring. |
| Test Example 2 | glycerin | 2.0 | S100 cannot be dissolved with overnight stirring and the mixture forms a semisolid. |

Results: since ethanol can dissolve the largest amount of phospholipids, and the other two solvents can only dissolve a small amount of phospholipids, which shows that phospholipids cannot be dissolved with overnight stirring. The prepared preparation has a viscosity of more than 300 cp or it is even in a semisolid form, which cannot be injected. It can be seen that when the same amount of solvent is used, ethanol has a better dissolving property than other solvents, can better dissolve the components in the preparation, and is easy to be administered by injection.

### Experimental Example 3

Different ratios of phospholipid Span 80 sustained release preparations were prepared. Local irritation experiments were carried out to investigate the irritating effects of different amount of anhydrous ethanol on the injection site, including phenomena such as red swelling and ulceration.

The results are shown in the following table:

| Prescription | Phospholipid S100 (g) | Span 80 (g) | Ethanol (g) | Results of local irritation experiments |
|---|---|---|---|---|
| Prescription one | 5.0 | 4.0 | 1.0 | No obvious irritation at the site of injection |
| Prescription two | 5.0 | 3.0 | 2.0 | No obvious irritation at the site of injection |
| Prescription three | 5.0 | 2.0 | 3.0 | Slight red swelling at the site of injection, but it disappeared after 3-4 days. |
| Prescription four | 5.0 | 1.0 | 4.0 | Red swelling at the site of injection, and skin ulcerated after 4-5 days. |

Results: when the content of anhydrous ethanol in the preparation was less than 30%, the preparation was less irritating to the administration site. In theory, when the ethanol solution containing a certain amount of water is used instead of anhydrous ethanol, its toxicity should be lower.

### Experimental Example 4

### (1) Phospholipid Span gel phase transition process

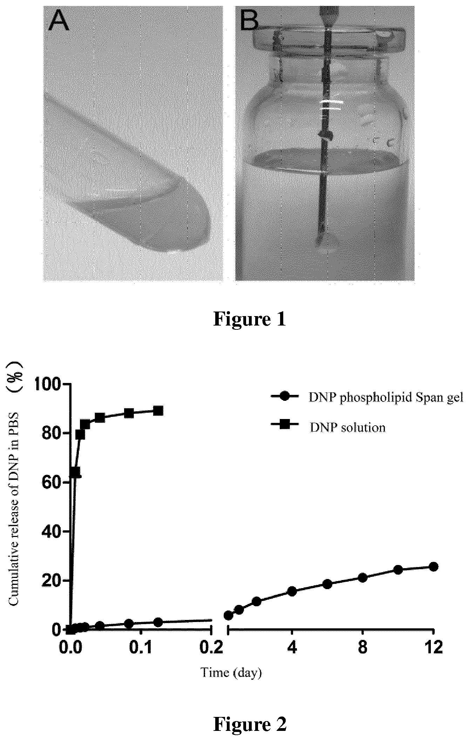

The product of Example 1 was poured into water. The result is shown in Figure 1. The phospholipid Span gel changed from a creamy yellow transparent liquid to a spherical semisolid state, indicating that a phase transition process occurred after the phospholipid Span gel was diffused.

### (2) In vitro release results

The product of Example 1 and solution group (Test Example 4 in Experimental Example 1) were separately placed in dialysis bags, and then they were placed in a 50mL of PBS buffer solution (pH=7.4), and an *in vitro* release test was carried out in a constant temperature oscillator (37 °C, 100 rpm). 5 ml of the buffer was taken at the setting time point and an equal volume of fresh PBS buffer was added. Calculate the cumulative release rate of 2,4-dinitrophenol. The results are shown in Figure 2. The phospholipid Span gel group has the characteristic of inhibiting burst release.

### (3) In vivo pharmacokinetic results

The product of Example 1 was injected subcutaneously into male SD rats, whose blood was taken at regular intervals. The content of 2,4-dinitrophenol in plasma was determined by high-performance liquid chromatography-mass spectrometry (LC-MS/MS), thereby investigating the sustained release effect thereof.

200-220g of SD rats were randomly divided into three groups, solution group (Test Example 4 in Experimental Example 1), phospholipid Span gel group (Example 1), and high-concentration phospholipid gel group (prepared according to patent CN102526753A). There were 6 rats in each group and they were injected subcutaneously at the dosage of 12.5 mg/kg. At the scheduled time point, blood was taken from the rat eye and placed in a heparin sodium anticoagulated test tube. After the sample was processed, the blood concentration was measured by LC-MS/MS injection.

The fomulation of the high-concentration phospholipid gel sustained release preparation (patent CN102526753A) is as follows:

| Ingredient | DNP | S100 | Medium chain triglyceride (MCT) | 85% ethanol |
|---|---|---|---|---|
| Dosage | 50mg | 7g | 1.5g | 1.5 g |

The pharmacokinetic parameters of each group of preparations are as follows:

| | Cmax (µg/mL) | Tmax (d) | t1/2 (d) | MRT (0→∞) (d) | AUC (0→∞) (mg/L*d) |
|---|---|---|---|---|---|
| Solution group | 54.48±5.12 | 0.02±0.02 | 0.11±0.01 | 0.17±0.01 | 10.19±1.11 |
| High-concentration phospholipid gel group | 33.56±4.11 | 0.04±0.03 | 0.2±0.33 | 0.24±0.11 | 11.33±0.78 |
| Phospholipid Span 80 gel group | 14.86±2.12 | 0.07±0.02 | 3.72±1.87 | 3.61±0.97 | 13.37±0.99 |

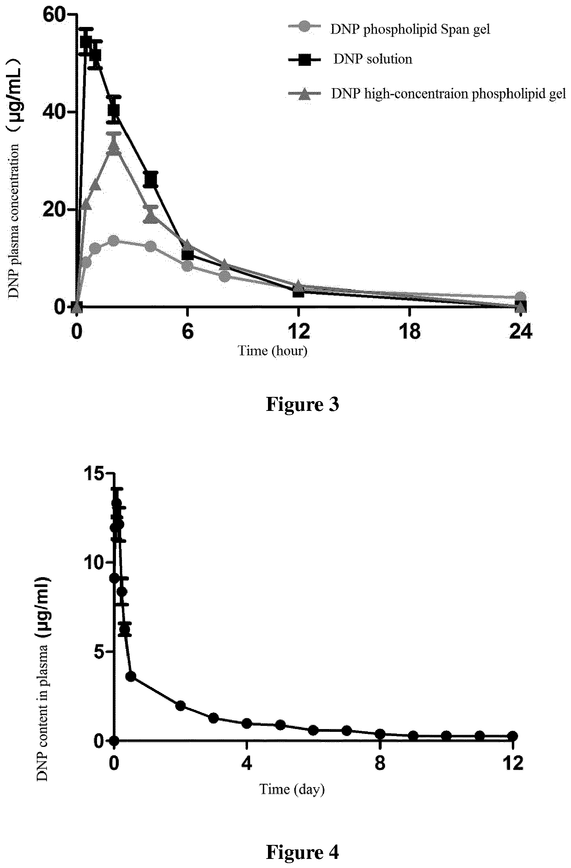

The results showed that the sustained release property of phospholipid Span gel was significantly better than that of high-concentration phospholipid gel and solution, mainly showing in a decrease in Cmax and a prolongation in t_{1/2}. As shown in Figures 3 and 4, the high-concentration phospholipid *in-situ* phase change gel sustained release preparation entrapping the small molecule drug DNP has a serious burst release, and the sustained release effect is not good, whereas the phospholipid Span gel preparation of the present disclosure can significantly reduce the burst release of the small molecule drug, and the sustained release effect is better.

### (3) Toxicity result

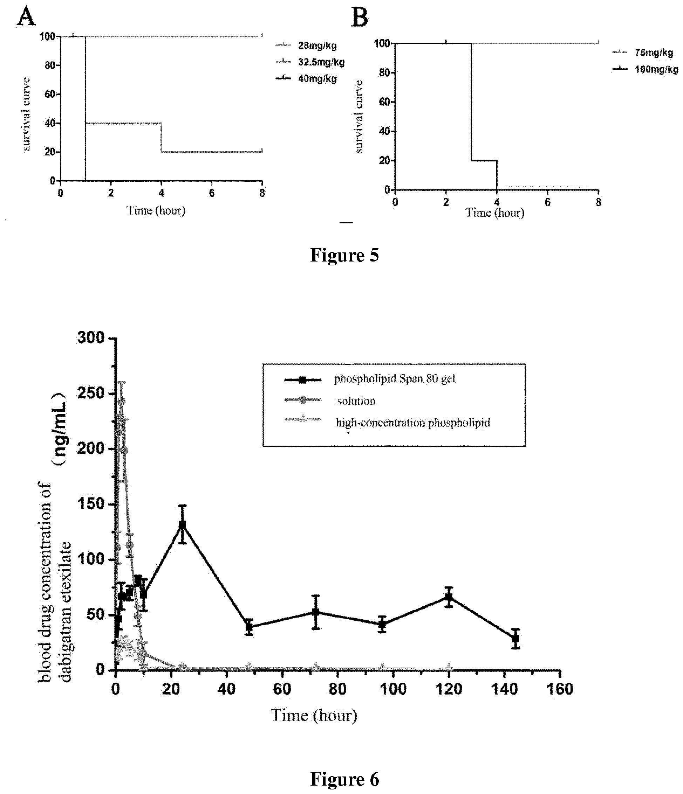

The product (B) of Example 1 and the drug solution (A) of the solution group (Test Example 4 in Experimental Example 1) in different drug doses were injected subcutaneously into male SD rats, and the death of the rats was observed and recorded. The results are shown in Figure 5, the gel preparation of the present disclosure can significantly reduce its side effects, which is because that the preparation has a good property of sustained release and inhibiting burst release. Compared with the solution group, the preparation can significantly reduce the blood drug concentration of DNP in the body, thereby improving its safety.

### Experimental Example 5

### (1) In vivo pharmacokinetic results

The product of Example 2 was injected subcutaneously into male SD rats, whose blood was taken at regular intervals, and the content of dabigatran etexilate in plasma was determined by high-performance liquid chromatography-mass spectrometry (LC-MS/MS), thereby investigating the sustained release effect thereof.

200-220g of SD rats were randomly divided into three groups, 6 rats in each group, and were administered subcutaneously with the following dosage: the solution group (11.4mg/kg; 200 mg of dabigatran etexilate was dissolved in 10 mL of phosphate buffer (pH=7.6), to prepare a solution of the same concentration as in Example 2), the phospholipid Span 80 gel group (80mg/kg) (Example 2), and the high-concentration phospholipid gel group (80mg/kg, gel was prepared according to patent CN102526753A but DNP in the high-concentration phospholipid gel group in Experimental Example 4 was replaced with 200mg dabigatran etexilate). At the scheduled time point, blood was taken from the rat eye and placed in a heparin sodium anticoagulated test tube. After the sample was processed, the blood concentration was measured by LC-MS/MS. The results showed that the sustained release ability of the phospholipid Span 80 gel group was significantly better than that of the high-concentration phospholipid gel and solution group, mainly showing in a prolongation in t_{1/2} and that dabigatran etexilate could maintain a high effective blood drug concentration. As shown in Figure 6, the high-concentration phospholipid *in-situ* phase change gel entrapping the small molecule drug dabigatran etexilate has a poor sustained release effect, whereas the phospholipid Span gel preparation of the present disclosure can significantly reduce the burst release of small molecule drugs, and the sustained release effect is better.

The pharmacokinetic parameters of each group of preparations are as follows:

| | Tₘₐₓ (h) | Cₘₐₓ (ng/mL) | t_{1/2} (h) | MRT₀₋ₜ (h) | AUC₀₋ₜ(ng/mL·h) |
|---|---|---|---|---|---|
| Solution group | 3.18 ± 0.21 | 243.86 ± 31.47 | 5.21± 0.83 | 7.86 ± 1.14 | 1966.75 ± 473.82 |
| High-concentration phospholipid gel group | 5.56±4.11 | 25.262±9.13 | 7.21±163 | 11.56 ± 0.34 | 200.33±50.69 |
| Phospholipid Span 80 gel group | 48.21 ± 3.56 | 131.95 ± 22.56 | 96.14± 8.72 | 138.42 ± 26.93 | 10774.2 ± 869.24 |

### (2) Local irritation experiment

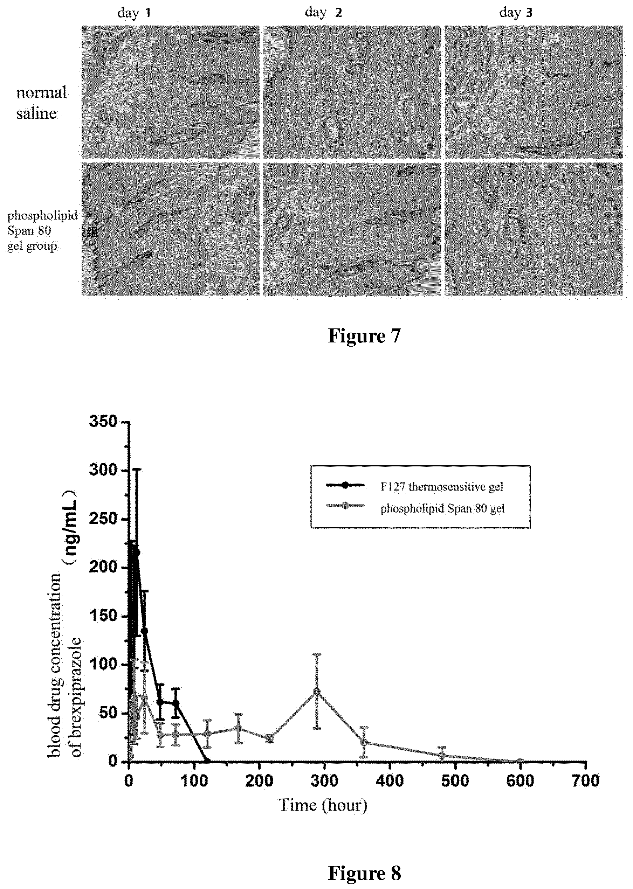

The product of Example 2 was injected subcutaneously into male SD rats, and on days 1, 14, and 21, the rats were sacrificed. The skin tissues were taken for HE staining. The results in Figure 7 indicate that the gel preparation has less irritation and good biocompatibility.

### Experimental Example 6

The product of Example 3 was injected subcutaneously into male SD rats, whose blood was taken at regular intervals. The content of brexpiprazole in plasma was determined by high-performance liquid chromatography-mass spectrometry (LC-MS/MS), which was compared with the F127 thermosensitive gel with significant sustained release effects (Lin Z,,et al. Novel thermo-sensitive hydrogel system with paclitaxel nanocrystals: High drug-loading, sustained drug release and extended local retention guaranteeing better efficacy and lower toxicity, Journal of Controlled Release 28 (2014) 161-70).

The fomulation of the F127 thermosensitive gel is as follows:

| Ingredient | Brexpiprazole | F127 | Water |
|---|---|---|---|
| Dosage | 200mg | 2g | 10g |

200-220g of SD rats were randomly divided into two groups, 6 rats in each group, and were injected subcutaneously with the following dosage: a phospholipid Span 80 gel group (50mg/kg) and a 20% F127 thermosensitive group (50mg/kg). At the scheduled time point, blood was taken from the rat eye and placed in a heparin sodium anticoagulated test tube. After the sample was processed, the blood concentration was measured by LC-MS/MS. The results showed that the sustained release ability of the phospholipid Span 80 gel group was significantly better than that of the F127 thermosensitive gel, mainly showing a prolongation in t_{1/2}. The brexpiprazole can maintain a highly effective blood drug concentration. As shown in Figure 8, although the F127 thermosensitive gel can sustained release for more than 100 hours, it was metabolized quickly and showed an obvious burst release, whereas the phospholipid Span gel preparation of the present disclosure had almost no drug burst release, and the sustained release effect was better, being able to continuously release for 25 days.

| | Tₘₐₓ (h) | Cₘₐₓ (ng/mL) | t_{0/2} (h) | MRT₀₋ₜ (h) | AUC₀₋ₜ(ng/mL·h) |
|---|---|---|---|---|---|
| F127 thermosensitive gel group | 12 | 215.7 | 22.4 | 14.8 | 51253.6 |
| Phospholipid Span 80 group | 24 | 74.9 | 66.5 | 31.0 | 91803.5 |

### Experimental Example 7

The sustained release properties of the phospholipid Span sustained release preparations for other small molecule drugs.

*In vivo* experiments in animals were used to investigate the effects of other small molecule drugs on inhibiting burst release. The drugs in the following table were used to prepare the preparations according to the prescription amount and the preparation method of Example 1. The obtained phospholipid Span sustained release preparation and original drug solution of the other small molecule drugs (each drug was formulated with water, ethanol or DMSO for injection, depending on the specific solubility) were injected subcutaneously into male SD rats, whose blood was taken at regular intervals. The Cₘₐₓ of the small molecule drugs in plasma was determined by high-performance liquid chromatography-mass spectrometry (LC-MS/MS), thereby investigating the effect on inhibiting burst release. The results are shown in the following table:

| Drug | Phospholipid Span sustained release preparation Cₘₐₓ(µg/mL) | Original drug Cₘₐₓ(µg/mL) |
|---|---|---|
| Aspirin | 14.56±3.12 | 52.24±5.08 |
| theophylline | 1.54±0.32 | 5.78±1.23 |
| Carbamazepine | 4.46±0.57 | 53.21±6.75 |
| Phenobarbital | 2.51±0.68 | 23.61±4.91 |
| Risperidone | 0.34±0.04 | 3.21±0..25 |
| Huperzine A | 1.33±0.22 | 13.87±2.86 |
| Metformin hydrochloride | 0.82±0.09 | 2.71±0.45 |
| Loratadine | 0.02±0.01 | 0.24±0.05 |
| Nimodipine | 1.51 ±0.67 | 10.21±1.14 |
| Hydroflumethiazido | 0.87±0.52 | 5.78±2.06 |
| Doxorubicin | 0.03±0.01 | 2.83±0.14 |
| Naproxen | 6.17±1.29 | 24.17±5.36 |
| Dexamethasone | 0.13±0.05 | 0.53±0.12 |

The results indicated that the phospholipid Span gel preparation of the present disclosure can significantly reduce the burst release of small molecule drugs.

In summary, the phospholipid Span *in-situ* phase change gel can significantly inhibit the burst release of small molecule drugs, prolong the release time, reduce the toxicity of small molecule drugs, and has good biocompatibility.

The above descriptions are only preferred embodiments of the present disclosure, and it should be noted that those skilled in the art can also make several improvements and modifications without departing from the principles of the present disclosure. These improvements and modifications should be considered to fall within the scope of protection of the present disclosure.

## Claims

1. An *in-situ* gel carrier, wherein it comprises a phospholipid, a Span, and an ethanol solution.

2. The *in-situ* gel carrier according to claim 1, wherein it comprises, in parts by weight, 30-60 parts of phospholipid, 10-40 parts of Span, and 7-30 parts of ethanol solution, wherein the ethanol solution has a concentration range of 70-100% (v/v).

3. The *in-situ* gel carrier according to claim 1 or 2, wherein the phospholipid is selected from one or more of a natural phospholipid, a semisynthetic phospholipid, and a synthetic phospholipid.

4. The *in-situ* gel carrier according to claim 3, wherein the natural phospholipid is selected from egg yolk lecithin and soybean lecithin; the semisynthetic phospholipid is selected from hydrogenated egg yolk lecithin and hydrogenated soybean lecithin; and the synthetic phospholipid is selected from dipalmitoyl phosphatidylethanolamine, dipalmitoyl phosphatidic acid, dipalmitoyl phosphatidylglycerole, dioleoyl phosphatidylethanolamine, dipalmitoyl phosphatidylcholine, distearoyl phosphatidylcholine, dimyristoyl phosphatidylcholine, preferably soybean lecithin S100.

5. The *in-situ* gel carrier according to claim 1 or 2, wherein the Span is selected from one or more of Span80, Span85, Span60, Span40, and Span20, preferably Span80.

6. The *in-situ* gel carrier according to claim 1 or 2, wherein the ethanol solution is selected from anhydrous ethanol, ethanol-water solution, ethanol-normal saline solution, ethanol-phosphate buffer solution, ethanol carbonate buffer solution, ethanol-succinate buffer solution, ethanol-citrate buffer solution, and ethanol-lactate buffer solution.

7. An *in-situ* gel preparation, wherein it comprises a phospholipid, a Span, an active pharmaceutical ingredient, and an ethanol solution.

8. The *in-situ* gel preparation according to claim 7, wherein it comprises, in parts by weight, 0.01-20 parts of active pharmaceutical ingredient, 30-60 parts of phospholipid, 10-40 parts of Span, and 7-30 parts of ethanol solution, wherein the ethanol solution has a concentration range of 70-100% (v/v).

9. The *in-situ* gel preparation according to claim 7 or 8, wherein the phospholipid is selected from one or more of a natural phospholipid, a semisynthetic phospholipid, and a synthetic phospholipid.

10. The *in-situ* gel preparation according to claim 9, wherein the natural phospholipid is selected from egg yolk lecithin and soybean lecithin; the semisynthetic phospholipid is selected from hydrogenated egg yolk lecithin and hydrogenated soybean lecithin; and the synthetic phospholipid is selected from dipalmitoyl phosphatidylethanolamine, dipalmitoyl phosphatidic acid, dipalmitoyl phosphatidylglycerole, dioleoyl phosphatidylethanolamine, dipalmitoyl phosphatidylcholine, distearoyl phosphatidylcholine, dimyristoyl phosphatidylcholine, preferably soybean lecithin S100.

11. The *in-situ* gel preparation according to claim 7 or 8, wherein the Span is selected from one or more of Span80, Span85, Span60, Span40, and Span20, preferably Span80.

12. The *in-situ* gel preparation according to claim 7 or 8, wherein the ethanol solution is selected from anhydrous ethanol, ethanol-water solution, ethanol-normal saline solution, ethanol-phosphate buffer solution, ethanol carbonate buffer solution, ethanol-succinate buffer solution, ethanol-citrate buffer solution, and ethanol-lactate buffer solution.

13. The *in-situ* gel preparation according to claim 7 or 8, wherein the active pharmaceutical ingredient is a fat-soluble drug.

14. The *in-situ* gel preparation according to claim 7 or 8, wherein the active pharmaceutical ingredient is selected from one or more of 2,4-dinitrophenol, dabigatran etexilate, aspirin, ridogrel, ticlopidine, clopidogrel, heparin, logiparin, lomoparin, warfarin, dicoumarol, theophylline, aminophylline, choline theophyllinate, salbutamol, clenbuterol, terbutaline, ipratropium bromide, sodium tryptophan, ketotifen, sodium valproate, carbamazepine, phenytoin sodium, ethosuximide, primidone, chloral hydrate, zopiclone, zaleplon, phenobarbital, amobarbital, sodium thiopental, diazepam, oxazepam, clonazepam, nitrazepam, triazolam, alprazolam, estazolam, glipizide, diltiazem, tramadol, morphine, pethidine, fentanyl, methadone, pentazocine, buprenorphine, codeine, naloxone, diclofenac, diclofenac sodium, ibudilast, ambroxol hydrochloride, oxycodone, chlorpromazine, trifluoperazine, fluphenazine, fluphenazine decanoate, fluphenazine enanthate, perphenazine enanthate, perphenazine, thioridazine, chlorprothixene , flupentixol decanoate, clopenthixlo, haloperidol, haloperidol decanoate, pipotiazine palmitate, trifluridol, pimozide, trichlorpromazine, thioridazine, fluspirilene, droperidol, clozapine, loxapine, clotiapine, olanzapine, quetiapine, sulpiride, sultopride, tiapride, tiapride, penfluridol, risperidone, morpholone, oxypertine, clomacran, reserpine, imipramine, amitriptyline, maprotiline, clomipramine, mianserin, sertraline, fluoxetine, fluvoxamine, citalopram, moclobemide, trazodone, lithium carbonate, tolterodine, levodopa, ziprasidone, levetiracetam, retigabine, perampanel, brivaracetam, eslicarbazepine, oxcarbazepine, duloxetine, paroxetine, vortioxetine, desmethylvenlafaxine, escitalopram, agomelatine, reboxetine, cariprazine, paliperidone, pramipexole, rasagiline, ropinirole, opicapone, safinamide, alfacalcidol, eldecalcitol, edaravone, aripiprazole, brexpiprazole, carbidopa, benserazide, selegiline, amantadine, memantine, bromocriptine, trihexyphenidyl, galantamine, huperzine A, rivastigmine, xanomeline, citicoline, piracetam, pyritinol, venlafaxine, bupropion, fluvastatin sodium, gliclazide, metformin, acyclovir, bezafibrate, fenofibrate, gemfibrozil, ciprofibrate, niacin, allopurinol, magnesium valproate, vincamine, promethazine, diphenhydramine, tripelennamine, chlorpheniramine, buclizine, phenindamine, cyproheptadine, hydroxyzine, cyclizine, meclizine, loratadine, cetirizine, efletirizine, metronidazole, clemastine, azelastine, acrivastine, mizolastine, astemizole, pheniramine, brompheniramine, tolpropamid, pyrrobutamine, triprolidine, nefopam, indapamide, tamsulosin, emedastine, oxybutynin, buflomedil, tamsulosin, propranolol, metoprolol, nadolol, pindolol, atenolol, alprenolol, acebutolol, bisoprolol, betaxolol, labetalol, carazolol, prazosin, captopril, enalapril, benazepril, fosinopril, cilazapril, trandolapril, alacepril, delapril, perindopril, quinapril, nifedipine, amlodipine, levoamlodipine, nimodipine, nicardipine, felodipine, lacidipine, nisoldipine, isradipine, verapamil, losartan, valsartan, telmisartan, irbesartan, candesartan, pomisaratan, terazosin, doxazosin, clonidine, moxonidine, reserpine, guanethidine, sodium nitroprusside, hydralazine, minoxidil, pinacidil, nicorandil, urapidil, piribedil, cicletanine, enalkiren, remikiren, hydrochlorothiazide, bendroflumethiazide, hydroflumethiazido, cyclopenthiazide, curcumin, tacrine, almitrine, platinum ligand, methotrexate, fluorouracil, thiopurine, tioguanine, hydroxyurea, cytarabine, everolimus, tacrolimus, acipimox, sirolimus, tegafur, pentostatin, gemcitabine, cyclophosphamide, busulfan, carmustine, dacarbazine, vinblastine, vincristine, vinorelbine, vindesine, paclitaxel, docetaxel, cabazitaxel, camptothecin, hydroxycamptothecin, teniposide, etoposide, irinotecan, topotecan, harringtonine, homoharringtonine, doxorubicin, pirarubicin, aclarubicin, idarubicin, epirubicin, daunorubicin, actinomycin D, plicamycin, clarithromycin, mitoxantrone, ibuprofen, acetaminophen, sulindac, fenoprofen, flurbiprofen, ketoprofen, meloxicam, piroxicam, nimesulide, rofecoxib, celecoxib, colchicine, probenecid, sulfinpyrazone, benzbromarone, phenylbutazone, mefenamic acid, clofenamic acid, naproxen, indomethacin, etodolac, magnesium salicylate, choline salicylate, salicylamide, salsalate, diflunisal, adrenocortical hormone, estrogen, androgen, tamoxifen, raloxifene, clomiphene, methyltestosterone, testosterone propionate, testosterone phenylacetate, medroxyprogesterone, megestrol, chlormadinone, hydroxyprogesterone acetate, hydrocortisone, cortisone, prednisone, prednisolone, methylprednisolone, dexamethasone, betamethasone, flutamide, retinoic acid, imatinib, gefitinib, erlotinib, thalidomide, lenalidomide, procarbazine, chlorambucil, melphalan or a pharmaceutically acceptable salt of the above medicaments.

15. The *in-situ* gel preparation according to claim 14, wherein the active pharmaceutical ingredient is a fat-soluble drug.

16. A method for preparing the *in-situ* gel preparation according to any one of claims 7 to 15, wherein the method comprises the following steps
(1) dissolving an active pharmaceutical ingredient in an appropriate amount of ethanol solution, filtering and sterilizing by a microporous membrane to form a drug solution; and
(2) mixing an injection grade phospholipid and a Span with the drug solution of step (1) under a sterile condition, stirring to completely dissolve the phospholipid, leaving to stand for a moment to remove bubbles in the preparation, and subpackaging and sealing.

17. A method for preparing the *in-situ* gel preparation according to any one of claims 7 to 15, wherein the method comprises the following steps
(1) subjecting an active pharmaceutical ingredient to a pharmaceutically common crystallization or pulverization under a sterile condition to prepare drug microparticles;
(2) mixing a prescription amount of phospholipid and Span with an ethanol solution under a sterile condition, stirring to completely dissolve the mixture to prepare a carrier solution; and
(3) mixing the drug microparticles of step (1) with the carrier solution prepared in step (2) uniformly under a sterile condition, leaving to stand for a moment to remove bubbles in the preparation, and subpackaging and sealing.

18. Use of the *in-situ* injection phase change gel sustained release preparation according to any one of claims 7 to 15 in the preparation of a small molecule-containing pharmaceutical preparation for inhibiting drug burst release and prolonging sustained release time.
